Europäisches Patentamt

(19) European Patent Office  (11) Numéro de publication: **0 145 536**

Office européen des brevets  **B1**

(12) **FASCICULE DE BREVET EUROPÉEN**

(51) Int. Cl.⁴: **C 12 M 1/16**

(45) Date de publication du fascicule du brevet:
**20.07.88**

(21) Numéro de dépôt: **84402145.1**

(22) Date de dépôt: **24.10.84**

(54) **Fermenteur pour milieux solides.**

(30) Priorité: **28.10.83 FR 8317272**

(43) Date de publication de la demande:
**19.06.85 Bulletin 85/25**

(45) Mention de la délivrance du brevet:
**20.07.88 Bulletin 88/29**

(84) Etats contractants désignés:
**AT BE CH DE FR GB IT LI LU NL SE**

(56) Documents cité:
**DE-C-953 691**
**FR-A-2 421 039**

(73) Titulaire: **Société pour l'Equipement des Industries Chimiques "SPEICHIM", Tour Winterthur 102, Terrasse Boieldieu, F-92085 Paris-la Defense Cédex 18 (FR)**

(72) Inventeur: **Daujat, Francis Michel Régis, 43, rue du Pas Saint- Maurice, F-92 150 Suresnes (FR)**
Inventeur: **Henry, Christian, 28, rue Saint- Lazare, F-75009 Paris (FR)**

(74) Mandataire: **Lepeudry- Gautherat, Thérèse, ARMENGAUD JEUNE CABINET LEPEUDRY 6, rue du Fg. St- Honoré, F-75008 Paris (FR)**

## Description

La présente invention est relative à un fermenteur tout particulièrement adapté aux milieux solides.

Les fermentations en milieu solide sont connues depuis longtemps : elles ont été surtout appliquées au stade artisanal pour la préparation d'aliments fermentés tels que du pain.

Depuis quelques années, ces techniques de fermentation en milieu solide font l'objet d'études afin de permettre leur application industrielle, en particulier dans un but d'économie d'énergie puisqu'en effet elles ne nécessitent pas, la concentration des produits obtenus en fin de fermentation. Comme applications de la fermentation en milieu solide, peuvent être citées à titre d'exemple la production d'aliments enrichis en protéines, d'enzymes, d'antibiotiques ou de métabolites.

Au Japon, des produits alimentaires ainsi que des enzymes sont obtenus industriellement par des procédés dérivés de ces procédés de fermentation traditionnelle : par exemple une amylase est produite selon le procédé dénommé "koji" qui est réalisé industriellement en couche mince dans des fermenteurs à plateaux.

La fermentation en milieu solide de produits ou de sous-produits provenant des industries agro-alimentaires, comporte généralement trois phases après le chargement de la matière première dans le fermenteur, à savoir :

a) Humidification puis cuisson à la vapeur du produit ou substrat de fermentation pour obtenir l'éclatement des parois cellulaires et la libération de l'amidon, suivies d'un refroidissement par un courant d'air puis de l'inoculation du substrat de fermentation à l'aide de spores du microorganisme choisi et de l'alimentation en sels minéraux nécessaires à sa croissance. Pendant toute cette phase, le produit est malaxé afin d'obtenir un milieu de fermentation parfaitement homogène ;

b) Incubation sous aération forcée avec de l'air humide saturé en eau, sous agitation périodique. Cette phase, également appelée phase de latence, correspond à la germination conduisant au développement du mycellium du microorganisme ;

c) Fermentation proprement dite qui est entièrement conduite sous le contrôle de la température qui déclenche des cycles périodiques de régulation c'est-à-dire de malaxage, d'apport d'eau, d'augmentation de l'aération forcée, et d'apport de solution pour corriger le pH.

Pour mettre en oeuvre un procédé de fermentation en milieu solide tel que défini ci-dessus, notamment lorsque le substrat de fermentation se présente ou peut être mis sous la forme de particules ou de granulés, on a pu utiliser des fermenteurs conçus pour travailler en couche profonde après en avoir augmenté la capacité. Ainsi le procédé d'enrichissement de produits amylacés en protéines décrit dans le brevet FR-A-2 353 232 peut être mis en oeuvre dans un fermenteur du type de celui décrit dans la demande FR-A-2 353 232.

Par ailleurs le brevet français 2 421 039 décrit un procédé de conditionnement microbiologique utilisant un fermenteur à alimentation continue qui prévoit une injection commune de l'air et de la vapeur d'eau au travers de la paroi inférieure de l'enceinte de fermentation.

Néanmoins l'utilisation de tels fermenteurs ne permet pas ou qu'imparfaitement la réalisation des différentes phases rappelées ci-dessus.

En effet la conception de ces fermenteurs ne permet pas de bien contrôler le déroulement de la fermentation en évitant les modifications ponctuelles. En particulier elle ne permet pas d'éviter le colmatage au niveau des injections de vapeur et d'avoir une répartition optimum des gaz injectés.

Aussi un des buts de la présente invention est-il de fournir un fermenteur pour milieu solide permettant de résoudre tous les problèmes technologiques posés par la mise en oeuvre industrielle de procédés de fermentation en milieu solide.

Un autre but de l'invention est de fournir un tel fermenteur qui peut s'adapter à toutes les capacités industrielles.

Ces buts ainsi que d'autres qui apparaîtront par la suite sont atteints par un fermenteur pour produits solides comprenant des dispositifs d'alimentation en produits solides, d'évacuation des produits issus de la fermentation et d'injection de fluides gazeux ou liquides, constitué par une enceinte d'axe vertical comportant une paroi inférieure et une paroi latérale munie d'une double enveloppe sur au moins une partie de sa hauteur ainsi qu'un mélangeur à axe vertical qui selon la présente invention est caractérisé en ce que la paroi inférieure est munie de dispositifs permettant d'injecter séparément de l'air, de la vapeur d'eau et un fluide de nettoiement.

L'enceinte comporte de préférene une paroi latérale cylindrique. Avantageusement elle comprend un volume inférieur délimité par la paroi inférieure et une plaque horizontale perforée pour laisser passer des liquides et des gaz, ce volume inférieur étant divisé par une cloison cylindrique de même axe vertical que l'enceinte, en une zone centrale tubulaire et en une zone annulaire.

De préférence, le diamètre de cette zone centrale tubulaire est égal à environ le cinquième du diamètre intérieur de l'enceinte.

Avantageusement chaque zone comporte un dispositif pour insuffler un gaz tel que de l'air ou pour injecter de la vapeur d'eau ou un fluide de nettoiement.

De préférence, l'insufflation de gaz et l'injection de vapeur d'eau ou du fluide de nettoiement s'effectuent par un même dispositif dans chaque zone.

Avantageusement le dispositif de nettoiement comporte dans chaque zone une évacuation.

De préférence, la paroi inférieure au niveau de la zone centrale tubulaire est munie d'un purgeur.

Selon un mode de réalisation avantageux de l'invention, la plaque horizontale est constituée d'un disque perforé recouvrant la zone centrale tubulaire et d'un disque annulaire recouvrant la zone annulaire. Ce dernier peut être constitué de plusieurs secteurs annulaires.

L'enceinte comprend, au niveau de la double enveloppe, un dispositif pour injecter de la vapeur d'eau au travers de la paroi latérale, dispositif constitué de préférence par un circuit en demi-coquille inclus dans cette double enveloppe. Un tel dispositif permet une injection supplémtntaire de vapeur d'eau au travers de la paroi latérale.

Avantageusement cette enceinte comporte à sa partie supérieure un pont tournant coopérant avec des moyens de support et de guidage situés à la périphérie de sa partie supérieure, ce pont tournant comportant le dispositif d'axe vertical pour mélanger les produits solides, lequel est excentré par rapport à l'axe de l'enceinte.

De préférence le dispositif d'axe vertical pour mélanger lesdits produits solides est constitué par une hélice à ruban hélicoïdal à pas progressif, qui est par exemple écrasé vers le bas.

Avantageusement, le fermenteur comprend un moteur pour entraîner cette hélice qui est située sur le pont tournant et un moteur pour entraîner ce pont tournant.

De préférence, ce pont tournant comporte des moyens pour injecter un liquide dans l'enceinte.

La description qui va suivre et qui ne présente aucun caractère limitatif, permettra à l'homme du métier d'être à même de réaliser la présente invention. Elle doit être lue en regard des figures annexées parmi lesquelles :

- la figure 1 représente en section verticale partielle, un fermenteur selon la présente invention ; et

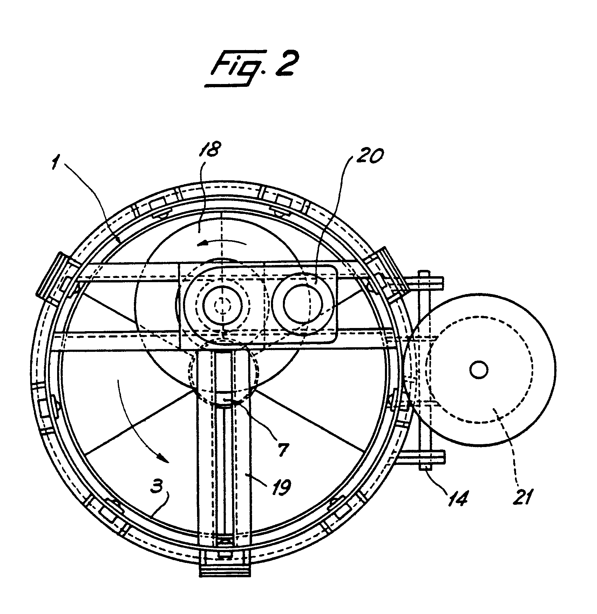

- la figure 2 est une vue de dessus de ce fermenteur selon la figure 1.

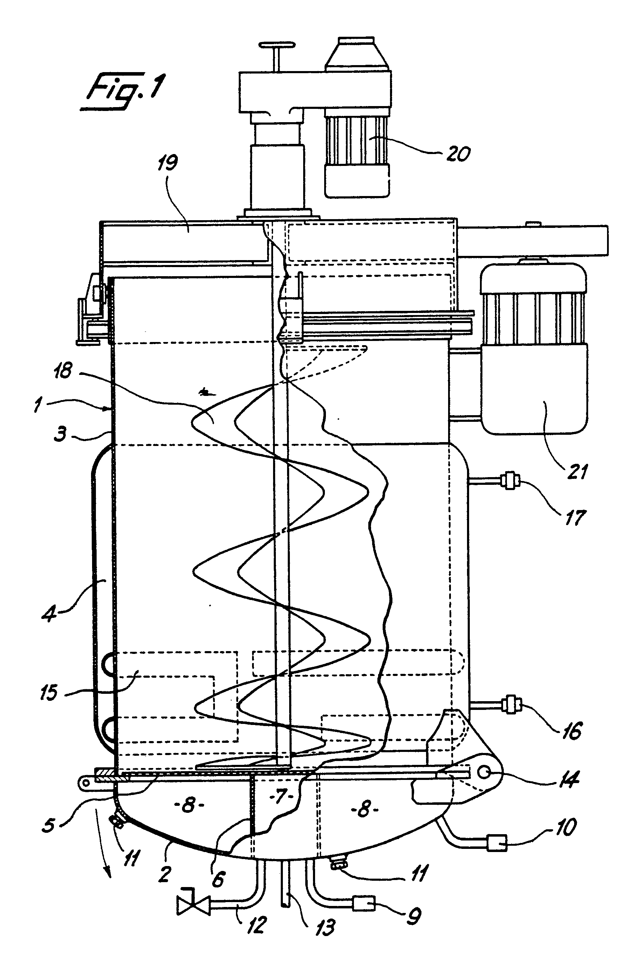

Ainsi qu'on peut le voir notamment sur la figure 1, un fermenteur pour produits solides selon la présente invention est constitué par une enceinte d'axe vertical désignée dans son ensemble par la référence 1. Cette enceinte comporte une paroi inférieure 2, et dans cet exemple de réalisation une paroi latérale 3 cylindrique munie d'une double enveloppe 4 sur au moins une partie de sa hauteur. Cette paroi latérale 3 pourrait éventuellement être constituée par plusieurs plans.

Cette enceinte 1 comprend des dispositifs pour insuffler un gaz au travers de sa paroi inférieure 2, des dispositifs pour injecter de la vapeur d'eau au travers de sa paroi inférieure 2 et de sa paroi latérale 3, un dispositif d'axe vertical pour mélanger les produits solides, ainsi que des systèmes de régulation et de nettoiement.

L'enceinte 1 comprend un volume inférieur délimité par sa paroi inférieure 2 et une plaque horizontale 5 perforée afin de laisser passer les gaz et les liquides. Ce volume est divisé par une cloison cylindrique 6 de même axe vertical que l'enceinte 1, en une zone centrale tubulaire 7 et en une zone annulaire 8.

Chaque zone 7 et 8 comporte un dispositif pour insuffler un gaz tel que de l'air, pour injecter de la vapeur d'eau et de nettoiement.

Selon le présent exemple de réalisation, ces dispositifs sont regroupés en un même dispositif pour chaque zone. Ainsi dans la zone centrale tubulaire 7 est situé un tel dispositif 9 et dans la zone annulaire 8 un tel autre dispositif 10. Ces dispositifs 9 et 10 permettent, à volonté, d'injecter de l'air, d'insuffler de la vapeur d'eau ou un fluide de nettoiement en fonction des phases du procédé de fermentation.

Au niveau des zones centrale tubulaire 7 et annulaire 8 sont disposés des systèmes, respectivement 11 et 12, pour l'évacuation des fluides de nettoiement.

Au niveau de la zone centrale tubulaire 7 peut également être disposé un purgeur 13 pour les éventuels condensats lors de la phase de préparation a) telle que décrite ci-dessus.

Quant à la plaque horizontale 5, elle est, selon cet exemple de réalisation, constituée d'un disque perforé recouvrant la zone centrale tubulaire 7 et d'un disque annulaire recouvrant la zone annulaire 8. Ce disque annulaire peut être constitué de plusieurs secteurs, par exemple au nombre de six.

Selon le cas, il peut être avantageux que la partie inférieure 2 de l'enceinte 1 puisse basculer autour d'un axe 14 situé sur la paroi latérale cylindrique 3: ceci permet notamment la vidange du produit issu de la fermentation par la partie basse de l'enceinte 1. L'alimentation en produits solides est, quant à elle, réalisée par tout dispositif connu: en particulier ceux-ci peuvent être directements introduits par le sommet de l'enceinte (1) qui n'est pas fermée.

Il est prévu un dispositif pour injecter de la vapeur d'eau au travers de la paroi latérale 3. Ce dispositif est constitué, selon cet exemple de réalisation, par un circuit en demi-coquilles 15 situé dans la double enveloppe 4. La vapeur qui circule dans les demi-coquilles 15 est injectée à l'intérieur de l'enceinte 1 à travers des perforations que comporte la paroi latérale 3 à ce niveau.

Quant à la double enveloppe 4, elle permet par une circulation d'eau de maintenir la température dans l'enceinte 1. Elle comporte une entrée d'eau 16 et une sortie d'eau 17.

Le dispositif d'axe vertical pour mélanger les produits solides est de préférence constitué par une hélice 18 qui est de type à ruban hélicoïdale à pas progressif, écrasé vers le bas de façon que le produit situé au-dessus de la plaque perforée 5 est repris par la partie basse, plus resserrée de l'hélice 18, et expansé durant son transfert vers la partie supérieure de l'enceinte 1.

Cette hélice 18 est montée sur un pont tournant 19 situé à la partie supérieure de l'enceinte 1 et coopérant avec des moyens de support et de guidage connus en eux-mêmes et disposés à la périphérie de cette partie supérieure. Elle est excentrée par rapport à l'axe vertical de l'enceinte 1.

Ce pont tournant 19 comporte un moteur 20 pour entraîner l'hélice 18 et est entraîné par un moteur 21 de façon connue en soi.

Par la double rotation de l'hélice 18 et du pont tournant 19, sont ainsi assurés l'homogénéisation et le brassage vertical du produit. Du fait des deux moteurs 20 et 21 entraînant respectivement l'hélice 18 et le pont tournant 19, on peut en fonction des applications régler séparément la vitesse de rotation de l'hélice 18 autour de son axe et la vitesse de déplacement de l'axe de l'hélice dans l'enceinte 1. Le même résultat pourrait être obtenu en utilisant qu'un seul de ces moteurs.

Par une telle disposition, on assure la circulation du produit du bas vers le haut de l'enceinte ainsi qu'un effet de détassage qui améliore la circulation de l'air et sa mise en contact avec le produit.

Le pont tournant 19 est par ailleurs équipé de buses d'aspersion constituant des moyens pour injecter dans l'enceinte 1, un liquide tel que, par exemple, de l'eau ou des solutions nutritives pour le ou les micro-organismes utilisés pour la fermentation.

Un tel fermenteur permet de réaliser les trois phases des procédés de fermentation telles que décrites ci-dessus.

## Revendications

1. Fermenteur pour produite solides comprenait des dispositifs d'alimentation en produite solides, d'évacuation des produite issus de la fermentation et d'injection de fluides qazeux ou liquides, constitué par une enceinte (1) d'axe vertical comportant une paroi inférieure (2) et une paroi latérale (3) munie d'une double enveloppe (4) sur au moins une partie de sa hauteur ainsi qu'un mélangeur à axe vertical, caractérisé en ce que la paroi inférieure (2) est munie de dispositifs permettant d'injecter séparément de l'air, de la vapeur d'eau et un fluide de nettoiement.

2. Fermenteur selon la revendication 1, caractérisé en ce que l'enceinte (1) comporte une paroi latérale (3) cylindrique.

3. Fermenteur selon la revendication 1 ou 2, caractérisé en ce que l'enceinte comprend un volume inférieur délimité par la paroi inférieure (2) et une plaque horizontale (5) perforée pour laisser passer des liquides et des gaz, ce volume inférieur étant divisé par une cloison cylindrique (6) de même axe vertical que l'enceinte (1) en une zone centrale tubulaire (7) et en une zone annulaire (8).

4. Fermenteur selon la revendication 3, caractérisé en ce que chaque zone est munie d'un dispositif pour insuffler soit de l'air, soit de la vapeur d'eau, soit un fluide de nettoiement.

5. Fermenteur selon la revendication 3 ou 4, caractérisé en ce que chaque zone comporte une évacuation (11, 12).

6. Fermenteur selon l'une quelconque des revendications 3 à 5, caractérisé en ce que la paroi inférieure au niveau de la zone centrale tubulaire (7) est munie d'un purgeur (13).

7. Fermenteur selon l'une quelconque des revendications 3 à 6, caractérisé en ce que la plaque horizontale (5) est constituée d'un disque perforé recouvrant la zone centrale tubulaire (7) et d'un disque annulaire recouvrant la zone annulaire (8).

8. Fermenteur selon la revendication 7, caractérisé en ce que le disque annulaire est constitué de plusieurs secteurs annulaires.

9. Fermenteur selon l'une quelconque des revendications 1 à 8, caractérisé en ce que l'enceinte (1) comprend un dispositif constitué par un circuit en demi-coquilles (15) permettant une injection supplémentaire de vapeur d'eau au travers de la paroi latérale (3).

10. Fermenteur selon l'une quelconque des revendications 1 à 9, caractérisé en ce que l'enceinte (1) comporte à sa partie supérieure un pont tournant (19) qui coopère avec des moyens de support et de guidage situés à la périphérie de ladite partie supérieure et sur lequel est monté un mélangeur à axe vertical excentré par rapport à l'axe de l'enceinte (1).

11. Fermenteur selon la revendication 10, caractérisé en ce que le mélangeur à axe vertical est constitué par une hélice à ruban hélicoïdale à pas progressif (18).

12. Fermenteur selon la revendication 11, caractérisé en ce que l'hélice à ruban hélicoïdale à pas progressif (18) est écrasée vers le bas.

13. Fermenteur selon l'une quelconque des revendications 10 à 12, caractérisé en ce qu'il comprend un moteur (20) pour entrainer l'hélice (18) qui est situé sur le pont tournant (19) et un moteur (21) pour entrainer le pont tournant (19).

14. Fermenteur selon l'une quelconque des revendications 10 à 13, caractérisé en ce que le pont tournant (19) comporte des moyens pour injecter un liquide dans l'enceinte (1).

15. Fermenteur selon l'une quelconque des revendications 1 à 14, caractérisé en ce que l'évacuation des produits de fermentation se fait par basculement de la paroi inférieure (2) autour d'un axe (14) situé sur la paroi latérale cylindrique (3).

## Patentansprüche

1. Fermenter für feste Produkte, der Vorrichtungen zur Zuführung fester Produkte, zur Entfernung der während der Fermentation gebildeten Produkte und zum Einblasen gasförmiger oder flüssiger Fluide aufweist und aus einem abgegrenzten Raum (1) mit einer senkrechten Achse sowie einer unteren Wand (2) und einer Seitenwand (3), die wenigstens in einem Abschnitt ihrer Höhe mit einem Doppelmantel (4) ausgerüstet ist, besteht, sowie mit einer Mischvorrichtung mit senkrechter Achse ausgestattet ist, dadurch gekennzeichnet, daß die untere Wand mit Vorrichtungen versehen ist, die ein separates Einblasen von Luft, Wasserdampf und eines Reinigungsfluids erlauben.

2. Fermenter nach Anspruch 1, dadurch gekennzeichnet, daß der abgegrenzte Raum (1) eine zylindrische Seitenwand (3) aufweist.

3. Fermenter nach Anspruch 1 oder 2, dadurch gekennzeichnet, daß der abgegrenzte Raum einen unteren Raum enthält, der durch die untere Wand (2) und eine durchlöcherte horizontale Platte (5) zum Durchlassen von Flüssigkeiten und Gasen begrenzt ist, wobei dieser untere Raum durch eine zylindrische Trennwand (6) mit derselben senkrechten Achse wie der des abgegrenzten Raums (1) in eine rohrförmige Mittelzone (7) und eine Ringzone (8) aufgeteilt ist.

4. Fermenter nach Anspruch 3, dadurch gekennzeichnet, daß jede Zone eine Vorrichtung zur Zuführung von Luft, Wasserdampf und eines Reinigungsfluids aufweist.

5. Fermenter nach Anspruch 3 oder 4, dadurch gekennzeichnet, daß jeder Bereich eine Ableitung (11, 12) aufweist.

6. Fermenter nach einem der Ansprüche 3 bis 5, dadurch gekennzeichnet, daß die untere Wand im Bereich der rohrförmigen Mittelzone (7) eine Ablaßvorrichtung (13) aufweist.

7. Fermenter nach einem der Ansprüche 3 bis 6, dadurch gekennzeichnet, daß die horizontale Platte (5) aus einer durchlöcherten Scheibe, welche die rohrförmige Mittelzone (7) bedeckt, und aus einer ringförmigen Scheibe, welche die Ringzone (8) bedeckt, besteht.

8. Fermenter nach Anspruch 7, dadurch gekennzeichnet, daß die ringförmige Scheibe aus mehreren ringförmigen Abschnitten besteht.

9. Fermenter nach einem der Ansprüche 1 bis 8, dadurch gekennzeichnet, daß der abgegrenzte Raum (1) eine Vorrichtung aufweist, die aus einer Ringleitung in Form von Halbschalen (15) besteht, die eine zusätzliche Zuführung von Wasserdampf durch die Seitenwand (3) erlaubt.

10. Fermenter nach einem der Ansprüche 1 bis 9, dadurch gekennzeichnet, daß der abgegrenzte Raum (1) in seinem oberen Bereich eine Drehbühne (19) aufweist, die mit am Umfang dieses oberen Bereichs angeordneten Träger- und Führungsmitteln zusammenwirkt und auf der ein Mischer mit senkrechter Achse vorgesehen ist, die zur Achse des abgegrenzten Raumes (1) exzentrisch angeordnet ist.

11. Fermenter nach Anspruch 10, dadurch gekennzeichnet, daß der Mischer mit senkrechter Achse aus einer durch ein Band gebildeten Schraube mit progressivem Schraubengang (18) besteht.

12. Fermenter nach Anspruch 11, dadurch gekennzeichnet, daß die durch ein Band gebildete Schraube mit progressivem Schraubengang (18) nach unten zusammengedrückt ist.

13. Fermenter nach einem der Ansprüche 10 bis 12, dadurch gekennzeichnet, daß er einen Motor (20) für den Antrieb der Schraube (18), der auf der Drehbühne (19) angeordnet ist, und einen Motor (21) zum Antrieb der Drehbühne (19) aufweist.

14. Fermenter nach einem der Ansprüche 10 bis 13, dadurch gekennzeichnet, daß die Drehbühne (19) Mittel zum Einblasen einer Flüssigkeit in den abgegrenzten Raum (1) aufweist.

15. Fermenter nach einem der Ansprüche 1 bis 14, dadurch gekennzeichnet, daß zur Entfernung der Fermentationsprodukte die untere Wand (2) um eine Achse (14), die an der zylindrischen Seitenwand (3) liegt, schwenkbar ist.

**Claims**

1. A fermenter for solid products comprising devices for feeding solid products, for removing the products following fermentation and for injecting gaseous fluids or liquids, formed by an enclosure (1) with vertical axis having a lower wall (2) and a side wall (3) with a double skin (4) over at least a part of its height as well as a mixer of vertical axis, characterized in that the lower wall (2) is provided with devices for separately injecting air, steam and a cleaning fluid.

2. The fermenter according to claim 1, characterized in that the enclosure (1) includes a cylindrical side wall (3).

3. Fermenter according to claim 1 or 2, characterized in that the enclosure comprises a lower volume defined by the lower wall (2) and a horizontal plate (5) perforated so as to let liquids and gases pass therethrough, this lower volume being divided by a cylindrical dividing wall (6) with the same vertical axis as the enclosure, (1) into a tubular central zone (7) and an annular zone (8).

4. Fermenter according to claim 3, characterized in that each zone is provided with a device for blowing in either air or steam or a cleaning fluid.

5. Fermenter according to claim 3 or 4, characterized in that each zone has a discharge means (11, 12).

6. Fermenter according to any one of claims 3 to 5, characterized in that the lower wall at the level of the central tubular zone (7) is provided with a purger (13).

7. Fermenter according to any one of claims 3 to 6, characterized in that the horizontal plate (5) is formed by a perforated disk covering the central tubular zone (7) and an annular disk covering the annular zone (8).

8. Fermenter according to claim 7, characterized in that the annular disk is formed of several annular sectors.

9. Fermenter according to any one of claims 1 to 8, characterized in that the enclosure (1) comprises a device formed by a circuit in the form of half shells (15) allowing additional injection of steam through the side wall (3).

10. Fermenter according to any one of claims 1 to 9, characterized in that the enclosure (1) has at its upper part a turning bridge (19) cooperating with support and guide means situated at the periphery of said upper part, and on which is mounted a mixer with vertical axis offcentered with respect to the axis of the enclosure (1).

11. Fermenter according to claim 10, characterized in that the mixer with vertical axis is formed by a screw with progressive pitch helical metal strip (18).

12. Fermenter according to claim 11, characterized in that the progressive pitch helical metal strip screw (18) is crushed towards the bottom.

13. Fermenter according to any one of claims 10 to 12, characterized in that it includes a motor (20) for driving the screw (18) which is situated on the turning bridge (19) and a motor (21) for driving the turning bridge (19).

14. Fermenter according to any one of claims 10 to 13, characterized in that the turning bridge (19) includes means for injecting a liquid into the enclosure (1).

15. Fermenter according to any one of claims 1 to 14, characterized in that removal of the fermentation products is provided by tilting the lower wall (2) about a shaft (14) situated on the cylindrical side wall (3).

*Fig.1*

*Fig. 2*